# EUROPÄISCHE PATENTANMELDUNG

(11) **EP 3 155 959 A1**
(43) Veröffentlichungstag der Anmeldung: **19.04.2017**
(21) Anmeldenummer: 16002176.2
(22) Anmeldetag: 05.10.2016
(51) Int. Cl.: A61B 5/00, A61B 5/103, A61B 5/11, A47C 7/62, A47C 9/00, A63B 21/00, A63B 23/02

(54) **VERFAHREN UND VORRICHTUNG ZUR DURCHFÜHRUNG GYMNASTISCHER ÜBUNGEN**

(30) Priorität: 09.10.2015 DE 102015013265
(71) Anmelder: Hübner Burkhard, 12524 Berlin (DE)
(72) Erfinder: Hübner Burkhard, 12524 Berlin (DE)
(74) Vertreter: Kietzmann, Manfred

(57) **Zusammenfassung**

Vorgeschlagen werden ein Verfahren und eine Vorrichtung zur Durchführung gymnastischer Übungen durch eine auf einem Sitzmöbel (1) auf einer Druckmessmatte oder -folie (2) sitzenden Person, wobei die Druckmessmatte oder -folie (2) über eine Vielzahl von beabstandet zueinander angeordneten Druckmesssensoren zur Feststellung des von der sitzenden Person ausgeübten Druckes auf die Druckmessmatte oder -folie (2) verfügt. Die Lage der Drucksensoren zueinander wird in einem Koordinatensystem erfasst und aus den sich entsprechend der jeweiligen Sitzhaltung verändernden Messwerten der Druckmesssensoren werden in zeitlichen Abständen jeweils ein Druckschwerpunkt (9) berechnet und die Koordinaten dieses Druckschwerpunktes (9) bestimmt. Die Koordinaten des jeweiligen Druckschwerpunktes (9) werden auf ein Koordinatensystem eines Bildschirmes (7) eines Rechners übertragen und zur Anzeige gebracht. Die Druckschwerpunkte (9) können zur Rechnersteuerung genutzt werden.

## Beschreibung

Die Erfindung betrifft ein Verfahren und eine Vorrichtung zur Durchführung gymnastischer Übungen im Sitzen unter Verwendung eines Datenverarbeitungsgerätes mit Bildschirm. Dabei kann das Vollziehen der gymnastischen Übungen direkt mit der Arbeit am PC verbunden sein.

Bekannt sind ergometrisch geformte Sitzmöbel, um bestimmte Sitzpositionen zu fördern. Dazu gehören z. B. mit einem Fluid gefüllte Sitzkissen (DE 21 2006 000 073 U1) oder Ballhocker (DE 20 2007 018 810 U1).

Ferner sind Massagestühle (DE 20 2009 005 074 U1) bekannt, die auf den Rücken und die Halswirbel des Sitzenden einwirken.

Weiter ist es bekannt, die Füße auf bewegbaren Plattformen zu positionieren, um so mit den Beinen gymnastische Übungen zu vollziehen (DE 20 2009 013 057 U1).

Diese Vorrichtungen sind nutzbar, ohne dass Arbeitstätigkeiten des Sitzenden unterbrochen werden müssen. Zwischen den gymnastischen Übungen und der Tätigkeit besteht aber Parallelität, d. h. sie erfolgen unabhängig voneinander.

Weiter ist es aus der DE 20 2009 000 999 U1 bekannt, das ein Trainierender bei der automatisierten Durchführung von Fitnesstrainingskursen über einen Bildschirm visuelle Anweisungen für das Training erhält.

Darüber hinaus sind technische Lösungen bekannt, die vom menschlichen Körper beim Stehen oder Sitzen ausgehende Druckbelastungen erfassen und zur Anzeige bringen.

So wird die Druckmessung in der DE 20 2006 008 296 U1 zur Ermittlung des Sitzknochenabstandes genutzt.

Die Druckmessmatte der DE 10 2007 030 583 A1 dient zur Feststellung der Rückenbelastung eines Pferdes durch Reiter und Sattel.

Das Messen der Druckverteilung an der Fußsohle wird in dem DE 295 12 711 U1 genutzt, um Überbelastungen mittels der Orthopädieschuhtechnik entgegenzuwirken.

Aufgabe der Erfindung ist es, eine Möglichkeit zu schaffen, die Arbeitstätigkeit mit der Durchführung von gymnastischen Übungen zu verbinden.

Gelöst wird diese Aufgabe mit dem Verfahren nach Anspruch 1 und einer Vorrichtung gemäß Anspruch 9. Vorteilhafte Ausgestaltungen sind Gegenstand der Unteransprüche.

Das erfindungsgemäße Verfahren zur Durchführung gymnastischer Übungen durch eine auf einem Sitzmöbel auf einer Druckmessmatte oder -folie sitzenden Person, wobei die Druckmessmatte oder -folie über eine Vielzahl von beabstandet zueinander angeordneten Druckmesssensoren zur Feststellung des von der sitzenden Person ausgeübten Druckes auf die Druckmessmatte oder -folie verfügt, sieht vor, dass die Lage der Drucksensoren zueinander in einem Koordinatensystem erfasst wird, aus den sich entsprechend der jeweiligen Sitzhaltung verändernden Messwerten der Druckmesssensoren in zeitlichen Abständen jeweils ein Druckschwerpunkt berechnet und die Koordinaten dieses Druckschwerpunktes bestimmt werden,
die Koordinaten des jeweiligen Druckschwerpunktes auf ein Koordinatensystem eines Bildschirmes eines Rechners übertragen und zur Anzeige gebracht werden.

Um die angezeigten Druckschwerpunkte mit der konkreten Arbeit am Rechner zu verbinden, sieht eine vorteilhafte Ausgestaltung vor, dass das Koordinatensystem des Bildschirmes sichtbare Steuerbuttons für den Rechner aufweist, die softwaremäßig mit den Anzeigen der Druckschwerpunkte gekoppelt sind, derart, dass beim Zusammenfallen der Anzeige eines Druckschwerpunktes mit einem Steuerbutton durch ein von der sitzenden Person generierbares Steuersignal der jeweilige Steuerbutton aktiviert wird.

Das Steuersignal wird bei einer bevorzugten Ausführung generiert aus einer erfassten und rechentechnisch verarbeiteten Sprachausgabe und/oder Bewegung eines Körperteils der sitzenden Person. Bevorzugt wird die Bewegung einer Wade oder eines Fußes der sitzenden Person zur Generierung des Steuersignals erfasst.

Eine weitere vorteilhafte Ausgestaltung des Verfahrens sieht vor, dass die in zeitlichen Abständen ermittelten und auf dem Bildschirm angezeigten Druckschwerpunkte gespeichert und entsprechend ihrer zeitlichen Reihenfolge miteinander verbunden und angezeigt werden. So entsteht ein Diagramm der Druckschwerpunkte über die Zeit, das auswertbar ist.

Dabei kann es von Vorteil sein, wenn die zeitlichen Abstände zur Ermittlung der Druckschwerpunkte einstellbar sind.

Eine weitere vorteilhafte Ausgestaltung sieht vor, dass auf dem Bildschirm gesundheitsfördernde Druckschwerpunktfolgen auf Abruf angezeigt werden. Die sitzende Person kann so angehalten werden, dieser Druckschwerpunktfolge durch die Veränderung der eigenen Sitzhaltung zu folgen.

Vorteilhafterweise sind derartige Druckschwerpunktfolgen zusammen mit den von der sitzenden Person durch Bewegung erzielten Druckschwerpunkten als vergleichende Diagramme ausdruckbar und können ebenfalls einer Auswertung unterzogen werden.

Eine erfindungsgemäße Vorrichtung zur Durchführung des Verfahrens weist mindestens auf
- ein Sitzmöbel mit einer Druckmessmatte oder -folie mit einer Vielzahl von beabstandet zueinander in einem Koordinatensystem angeordneten Druckmesssensoren zur Messung der von einer auf der Druckmessmatte oder -folie sitzenden Person ausgeübten Druckmesswerte auf die Drucksensoren,
- eine Schnittstelle zur Übermittlung der Druckmesswerte der Druckmesssensoren an einen Rechner, wobei durch den Rechner in zeitlichen Abständen der Druckschwerpunkt aus der Summe der Druckmesswerte ermittelbar ist,
- einen Bildschirm zur Anzeige der vom Rechner ermittelten Druckschwerpunkte in einem Koordinatensystem und zur Anzeige von Steuerbuttons für den Rechner,
- eine Software zur Verknüpfung der Steuerbuttons mit der Anzeige der Druckschwerpunkte,
- Mittel zur Generierung eines Steuersignals für die Software durch die sitzende Person, derart, dass beim Zusammenfallen der Anzeige eines Druckschwerpunktes mit einem Steuerbutton durch ein von der sitzenden Person generierbares Steuersignal der jeweilige Steuerbutton aktivierbar ist und
- Mittel zur Übertragung des Steuersignals an den Rechner.

Dabei kann die Druckmessmatte oder -folie mit dem Sitzmöbel lösbar verbunden sein oder die Druckmessmatte oder -folie ist in die Sitzfläche des Sitzmöbels eingearbeitet.

Als Mittel zur Generierung des Steuersignals für die Software sind erfasste und rechentechnisch verarbeitet Sprachausgaben und/oder Bewegungen der sitzenden Person geeignet, letzteres kann z. B. durch mindestens einen an dem Sitzmöbel angeordneten Schalter oder Sensor vorgenommen werden, der bevorzugt funktechnisch mit dem Rechner verbunden ist.

Die Erfindung soll anhand der Zeichnung erläutert werden. Es zeigen:
- Fig. 1: die Anordnung der Druckmessmatte auf einem Sitzmöbel,
- Fig. 2: das Zusammenfallen eines Druckschwerpunktes mit einem Steuerbutton und
- Fig. 3: eine vorgegebene Druckschwerpunktfolge und deren Nachvollzug.

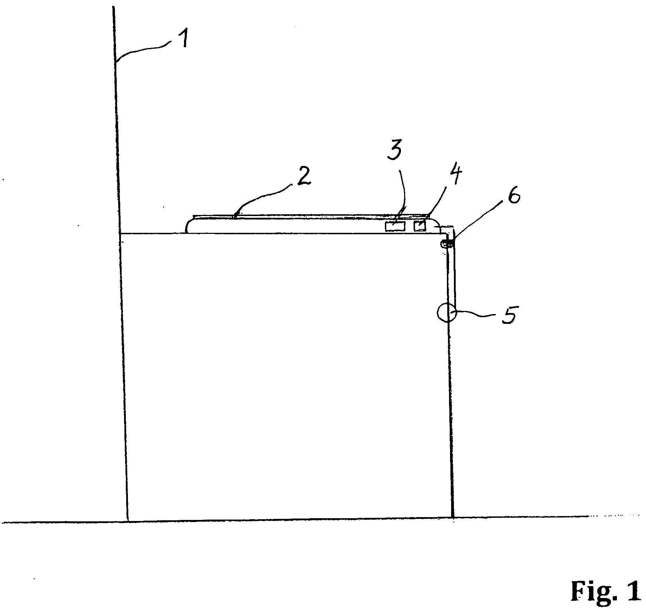

**Fig. 1** zeigt eine auf einem Sitzmöbel 1 angeordnete Druckmessfolie 2, die über eine Vielzahl von beabstandet zueinander angeordneten Druckmesssensoren zur Feststellung des von einer auf der Druckmessfolie 2 sitzenden Person ausgeübten Druckes auf die Druckmessfolie 2 verfügt. Die Lage der Druckmesssensoren ist in einem Koordinationssystem erfasst. Die Messwerte der Drucksensoren werden über eine Schnittstelle 3 zu einem Rechner übertragen, der in zeitlichen Abständen jeweils einen Druckschwerpunkt 9 berechnet. Dieser Druckschwerpunkt 9 wird jeweils auf einem Bildschirm 7 angezeigt. Dies kann mit oder ohne einemunterlegten Koordinatensystem vorgenommen sein.

Die zeitlichen Abstände der Anzeige des jeweiligen Druckschwerpunktes 9 können einstellbar sein, so dass auch kurzzeitige Veränderungen in der Sitzhaltung und damit einhergehend der Veränderung der Druckmesswerte der Drucksensoren anzeigbar sind.

Die Drucksensoren soweit erforderlich und die Schnittstelle 3 werden aus einer Batterie 4 mit Elektroenergie versorgt.

Weiter wird gezeigt, dass ein Sensor 5 an einem Bein des Sitzmöbels 1 mittels einer Klemme 6 angeordnet ist. Dieser Sensor 5 dient dazu, dass durch eine Beinbewegung der sitzenden Person ein Steuersignal generierbar ist.

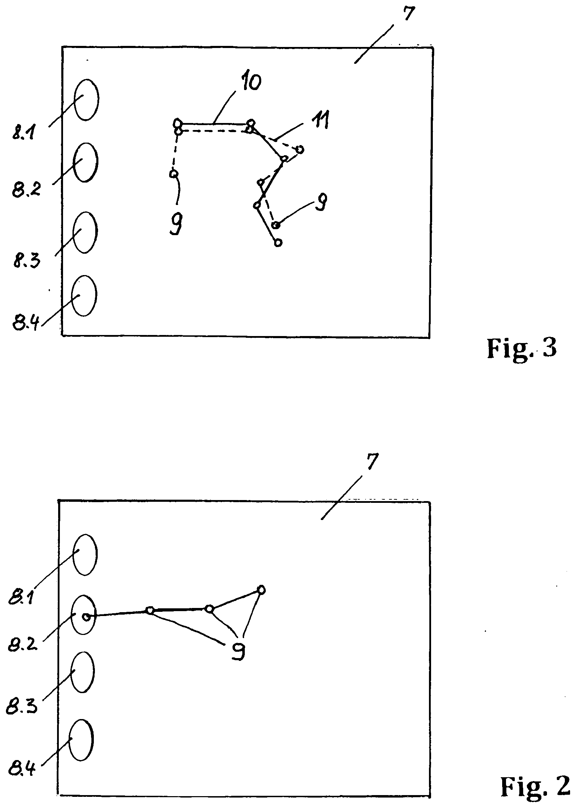

Erfindungsgemäß ist vorgesehen und wird in **Fig. 2** gezeigt, dass das Koordinatensystem des Bildschirmes sichtbare Steuerbuttons 8.1-8.n für den Rechner aufweist, die softwaremäßig mit den Anzeigen der Druckschwerpunkte 9 gekoppelt sind, derart, dass beim Zusammenfallen der Anzeige eines Druckschwerpunktes 9 mit einem Steuerbutton 8.2 durch ein von der sitzenden Person generierbares Steuersignal der jeweilige Steuerbutton aktivierbar ist. Damit wird die Funktion einer herkömmlichen Maus nachvollzogen. Werden beidseitig am Sitzmöbel 1 Sensoren 5 angeordnet, sind die Funktionen von rechter und linker Maustaste nachvollziehbar.

**Fig. 3** zeigt auf dem Bildschirm 7 eine vorgegebene aufrufbare Druckschwerpunktfolge 10. Die auf der Druckmessfolie 2 sitzende Person hat durch Gewichtsverlagerung versucht, diese Druckschwerpunktfolge 10 nachzuvollziehen. Dies sollen das Diagramm 11 der nachvollzogenen Druckschwerpunkte 9 zeigen (gestrichelte Linie). Für eine vergleichende Analyse lassen sich die beiden Diagramme ausdrucken und sind die ermittelten Druckschwerpunkte 9 in der zeitlichen Abfolge verbindbar. Die sitzende Person führt auf diese Weise für sie optimierte Bewegungen durch.

### Bezugszeichenliste

- 1: Sitzmöbel
- 2: Druckmessfolie
- 3: Schnittstelle
- 4: Batterie
- 5: Sensor
- 6: Klemmteil
- 7: Bildschirm
- 8.1-8.n: Steuerbutton
- 9: Druckschwerpunkte
- 10: vorgegebene Druckschwerpunktfolge
- 11: Diagramm nachvollzogene Druckschwerpunkte

## Patentansprüche

1. Verfahren zur Durchführung gymnastischer Übungen durch eine auf einem Sitzmöbel (1) auf einer Druckmessmatte oder -folie (2) sitzenden Person, wobei die Druckmessmatte oder -folie (2) über eine Vielzahl von beabstandet zueinander angeordneten Druckmesssensoren zur Feststellung des von der sitzenden Person ausgeübten Druckes auf die Druckmessmatte oder -folie (2) verfügt, **dadurch gekennzeichnet, dass**
die Lage der Drucksensoren zueinander in einem Koordinatensystem erfasst wird, aus den sich entsprechend der jeweiligen Sitzhaltung verändernden Messwerten der Druckmesssensoren in zeitlichen Abständen jeweils ein Druckschwerpunkt (9) berechnet und die Koordinaten dieses Druckschwerpunktes (9) bestimmt werden,
die Koordinaten des jeweiligen Druckschwerpunktes (9) auf ein Koordinatensystem eines Bildschirmes (7) eines Rechners übertragen und zur Anzeige gebracht werden.

2. Verfahren nach Anspruch 1, **dadurch gekennzeichnet, dass**
das Koordinatensystem des Bildschirmes (7) sichtbare Steuerbuttons (8.1-8.n) für den Rechner aufweist, die softwaremäßig mit den Anzeigen der Druckschwerpunkte (9) gekoppelt sind, derart, dass beim Zusammenfallen der Anzeige eines Druckschwerpunktes (9) mit einem Steuerbutton (8.n) durch ein von der sitzenden Person generierbares Steuersignal der jeweilige Steuerbutton (8.n) aktiviert wird.

3. Verfahren nach Anspruch 2, **dadurch gekennzeichnet, dass**
das Steuersignal durch eine erfasste und rechentechnisch verarbeitete Sprachausgabe und/oder Bewegung eines Körperteils der sitzenden Person generiert wird.

4. Verfahren nach Anspruch 3, **dadurch gekennzeichnet, dass**
die Bewegung einer Wade oder eines Fußes der sitzenden Person zur Generierung des Steuersignals erfasst wird.

5. Verfahren nach einem der Ansprüche 1 bis 4, **dadurch gekennzeichnet, dass**
die in zeitlichen Abständen ermittelten und auf dem Bildschirm angezeigten Druckschwerpunkte (9) gespeichert und entsprechend ihrer zeitlichen Reihenfolge miteinander verbunden und angezeigt werden.

6. Verfahren nach einem der Ansprüche 1 bis 5, **dadurch gekennzeichnet, dass**
die zeitlichen Abstände zur Ermittlung der Druckschwerpunkte (9) einstellbar sind.

7. Verfahren nach einem der Ansprüche 1 bis 6, **dadurch gekennzeichnet, dass**
auf dem Bildschirm gesundheitsfördernde Druckschwerpunktfolgen (10) auf Abruf angezeigt werden.

8. Verfahren nach Anspruch 7, **dadurch gekennzeichnet, dass**
die Druckschwerpunktfolgen (10) zusammen mit den von der sitzenden Person durch Bewegung erzielten Druckschwerpunkte (9) als vergleichende Diagramme (10, 11) ausdruckbar sind.

9. Vorrichtung zur Durchführung des Verfahrens nach einem der Ansprüche 1 bis 8 mindestens aufweisend
- ein Sitzmöbel (1) mit einer Druckmessmatte oder - folie (2) mit einer Vielzahl von beabstandet zueinander in einem Koordinatensystem angeordneten Druckmesssensoren zur Messung der von einer auf der Druckmessmatte oder -folie (2) sitzenden Person ausgeübten Druckmesswerte auf die Drucksensoren,
- eine Schnittstelle (3) zur Übermittlung der Druckmesswerte der Druckmesssensoren an einen Rechner, wobei durch den Rechner in zeitlichen Abständen der Druckschwerpunkt (9) aus der Summe der Druckmesswerte ermittelbar ist,
- einen Bildschirm (7) zur Anzeige der vom Rechner ermittelten Druckschwerpunkte (9) in einem Koordinatensystem und zur Anzeige von Steuerbuttons (8.1-8.n) für den Rechner,
- eine Software zur Verknüpfung der Steuerbuttons (8.1-8.n) mit der Anzeige der Druckschwerpunkte (9),
- Mittel zur Generierung eines Steuersignals für die Software durch die sitzende Person, derart, dass beim Zusammenfallen der Anzeige eines Druckschwerpunktes (9) mit einem Steuerbutton (8.n) durch ein von der sitzenden Person generierbares Steuersignal der jeweilige Steuerbutton (8.n) aktivierbar ist und
- Mittel zur Übertragung des Steuersignals an den Rechner.

10. Vorrichtung nach Anspruch 9, **dadurch gekennzeichnet, dass**
die Druckmessmatte oder -folie (2) mit dem Sitzmöbel (1) lösbar verbunden ist.

11. Vorrichtung nach Anspruch 9, **dadurch gekennzeichnet, dass**
die Druckmessmatte oder -folie (2) in die Sitzfläche des Sitzmöbels (1) eingearbeitet ist.

12. Vorrichtung nach einem der Ansprüche 9 bis 11, **dadurch gekennzeichnet, dass**
die Mittel zur Generierung des Steuersignals für die Software erfasste und rechentechnisch verarbeitet Sprachsignale oder Bewegungen der sitzenden Person sind.

13. Vorrichtung nach einem der Ansprüche 9 bis 11, **dadurch gekennzeichnet, dass**
die Mittel zur Generierung des Steuersignals mindestens ein an dem Sitzmöbel angeordneter Schalter oder Sensor (5) ist, der bevorzugt funktechnisch mit dem Rechner verbunden ist.

14. Vorrichtung nach einem der Ansprüche 9 bis 13, **dadurch gekennzeichnet, dass**
auf dem Bildschirm gesundheitsfördernde Druckschwerpunktfolgen (10) anzeigbar sind.
